# EUROPÄISCHE PATENTSCHRIFT

(11) **EP 4 302 738 B1**
(45) Veröffentlichungstag und Bekanntmachung des Hinweises auf die Patenterteilung: **11.06.2025**
(21) Anmeldenummer: 23181260.3
(22) Anmeldetag: 23.06.2023
(51) Int. Cl.: A61F 13/15, A61L 15/20

(54) **ABSORBIERENDER INKONTINENZARTIKEL ALS WEGWERFBARES EINMALPRODUKT UND VERFAHREN ZUR HERSTELLUNG DES INKONTINENZARTIKELS**
ABSORBENT INCONTINENCE ARTICLE AS DISPOSABLE PRODUCT AND METHOD OF MANUFACTURING THE INCONTINENCE ARTICLE
ARTICLE ABSORBANT POUR INCONTINENCE UTILISÉ COMME PRODUIT JETABLE, ET PROCÉDÉ DE FABRICATION D'UN ARTICLE POUR INCONTINENCE

(30) Priorität: 05.07.2022 DE 102022116721
(43) Veröffentlichungstag der Anmeldung: 10.01.2024
(73) Patentinhaber: Paul Hartmann AG, 89522 Heidenheim (DE)
(72) Erfinder: Bährle, Christian, 89542 Herbrechtingen (DE)
(74) Vertreter: DREISS Patentanwälte PartG mbB

(56) Entgegenhaltungen:
- US-A- 5 219 646
- US-B1- 6 428 900
- US-B2- 8 431 232

## Beschreibung

Die Erfindung betrifft einen absorbierenden Inkontinenzartikel als wegwerfbares Einmalprodukt und ein Verfahren zum Herstellen des Inkontinenzartikels umfassend ein flüssigkeitsdurchlässiges Topsheet, einen darunter angeordneten Absorptionskörper zur Aufnahme und dauerhaften Speicherung von flüssigen Körperausscheidungen, ein darunter zumindest in einem zentralen Bereich flüssigkeitsundurchlässig ausgebildetes Backsheet und vorzugsweise eine zwischen dem Topsheet und dem Absorptionskörper angeordnete Flüssigkeitsaufnahme- und - verteilerschicht, wobei wenigstens eine der vorgenannten Komponenten ein pH-Regulationsmittel mit Adipinsäure aufweist.

Typischerweise werden in Inkontinenzartikeln als pH-Regulationsmittel beispielsweise Zitronensäure oder Mononatriumzitrat eingesetzt. Sie werden entweder in Partikelform oder in gelöster Form in den Absorptionskörper des Inkontinenzartikels eingebracht. Die Einbringung in gelöster Form ist insofern aufwändig, als das verwendete Lösungsmittel wieder verdampft werden muss. Auch wenn als Lösungsmittel nur Wasser verwendet wird, so ist zum Verdampfen die Bereitstellung von Hitze erforderlich, was wiederum die Materialien des gesamten Produkts beeinträchtigen kann. Außerdem kann heißer Wasserdampf als für die Mitarbeiter gefährlich eingestuft werden. Verdampfendes Wasser führt in der Maschine zudem zu Korrosion, und ein Verdampfungsprozess ist bei hoher Maschinengeschwindigkeit kaum realisierbar, da er zu viel Zeit in Anspruch nimmt. Daher werden die genannten pH-Regulationsmittel üblicherweise in Partikelform oder als Granulat bereitgestellt und eingebracht. Doch auch dies stellt bei hohen Produktionsgeschwindigkeiten von typischerweise 200 bis 400 m/min eine große Herausforderung dar, wenn eine mengengenaue und zielgenaue Applikation des pH-Regulationsmittels gewünscht ist. Eine mengen- und zielgenaue Applikation ist aber gewünscht, um eine intendierte Wirkung und Effektivität der pH-Regulierung bei Einnässung des Inkontinenzartikels sicherzustellen. Es hat sich weiter gezeigt, dass für diese Art der Applikation nicht alle Korngrößen oder Korngrößenverteilungen verwendbar sind, was wiederum mitunter die kommerzielle Verfügbarkeit eines geeigneten pH-Regulationsmittels einschränkt.

Gemäß EP 2 491 910 A1 wurden pH-Regulationsmittel, wie Zitronensäure, als organischer Zusatz zu einem vernetzbaren Vinylacetate-Ethylene (VAE) Emulsionscopolymerlatex-Binder hinzugefügt und in eine Faserstruktur einer Flüssigkeitsaufnahme- und -verteilerschicht eingebracht.

WO 2021/160627 A1 offenbart einen Inkontinenzartikel in Höschenform, bei dem eine körperzugewandte Schicht oder Verteilerschicht eine Polycarbonsäure als pH-Regulationsmittel aufweist, wobei diese bevorzugt ausgewählt ist aus der Gruppe bestehend aus Zitronensäure, Isocitronensäure, Citraconsäure, Weinsäure, Itaconsäure, 1,2,3-Propantricarbonsäure, 1,2,3,4 Butantetracarbonsäure, Glutarsäure, Alpha-Ketoglutarsäure, Apfelsäure, Malonsäure, 2-Hydroxymalonsäure, Oxalsäure, Oxalosuccinsäure, Bernsteinsäure, Carboxybernsteinsäure, 1,2-Dimethylbernsteinsäure, Adipinsäure, Pimelinsäure, 2-Methyltricarballylsäure, Aconitsäure, 1,2,4 Butantricarbonsäure, Polymere der Acrylsäure und Mischungen davon, wobei die Säure vorzugsweise Zitronensäure ist. Das pH-Regulationsmittel wird dabei in Form einer Lösung eingebracht.

Gemäß DE 24 48 471 A1 sei festgestellt worden, dass verschiedene der in Frage kommenden Carbonsäuren, insbesondere die Zitronensäure, bei Befeuchten der Windel mit Urin zu leicht löslich seien, so dass das pH-Regulationsmittel zu leicht ausgewaschen werde mit der Folge, dass der zu den Rändern der Windel abfließende Urin relativ viel Säure enthalte, während der mittlere Teil der Windel nicht mehr gegen die Bildung von Ammoniak geschützt sei. Es komme hierdurch an den Rändern der Windel infolge der dort angereicherten Säure wegen eines sehr geringen pH-Werts zu Hautreizungen. Andere Carbonsäuren, beispielsweise Fumarsäure, lösten sich hingegen zu langsam auf, wobei das vorgenannte Problem dennoch bestehe, da es sich hierbei um relativ starke Säuren handle. Mit dieser Druckschrift wird vorgeschlagen, als pH-Regulationsmittel Adipinsäure zu verwenden. Die Säure könne auf vielerlei Weisen in den Hygieneartikel eingebracht werden, beispielsweise dispergiert in einen Träger, wie Wasser, Äthanol, und anschließendes Aufsprühen, Eintauchen oder Aufdrucken. Auch das Aufbringen in gelöstem Zustand oder in Form einer heißen Schmelze nach Erwärmen der Adipinsäure auf über 152°C ist erwähnt. Es wird weiter vorgeschlagen, Adipinsäure mit 1 bis 10 Gew.-% Azelainsäure zu kombinieren, um hierdurch die Heißwasserlöslichkeit zu steigern. Schwierigkeiten beim Einbringen von Adipinsäure in flüssiger Form in den Inkontinenzartikel könnten dadurch überwunden werden, dass die Säure in trockener Form als Granulat eingebracht wird.

US 6 428 900 Bl offenbart eine wasserempfindliche Schmelzklebstoffzusammensetzung auf Basis eines sulfonierten Polyestercopolymers. Die Zusammensetzung umfasst: (a) etwa 10 bis etwa 90 Gew.-% eines verzweigten sulfonierten Copolyester-Polymers oder einer Mischung aus verzweigten sulfonierten Copolyester- Polymeren, wobei das verzweigte sulfonierte Copolyester-Polymer ein Reaktionsprodukt eines multifunktionellen Reaktanten mit mindestens drei funktionellen Gruppen umfasst, ausgewählt aus Hydroxyl, Carboxyl und Mischungen davon, wobei mindestens ein Teil des multifunktionellen Reaktanten mindestens drei Hydroxylgruppen enthält; (b) etwa 5 bis etwa 50 Gew.-% eines Polyethylenglykols mit einem Molekulargewicht von mehr als 2000 und einem Schmelzpunkt von mehr als 50°C; (c) etwa 0 Gew.-% bis etwa 80 Gew.-% eines kompatiblen klebrigmachenden Harzes; und (d) etwa 0 Gew.-% bis etwa 40 Gew.-% eines Weichmachers; (e) etwa 0 bis etwa 3 Gew. -% eines Stabilisators wie z.B. Glycerin; und (f) wobei sich die Komponenten der Zusammensetzung auf 100 Gew.-% ergänzen und wobei die Klebstoffzusammensetzung die Integrität eines Einwegartikels, z.B. eines Inkontinenzartikels, während des normalen Gebrauchs aufrechterhält, sich jedoch in Gegenwart von Wasser zersetzt, wodurch ermöglicht wird, dass der Einwegartikel leicht zerlegt und recycelt werden kann.

Der vorliegenden Erfindung liegt die Aufgabe zugrunde, einen absorbierenden Hygieneartikel mit einem mengen- und zielgenau applizierten pH-Regulationsmittel zu schaffen, welcher in prozessstabiler Weise und unter wirtschaftlich vertretbaren Bedingungen hergestellt werden kann.

Diese Aufgabe wird erfindungsgemäß gelöst durch einen absorbierenden Inkontinenzartikel mit den Merkmalen des Anspruchs 1. Vorteilhafte Ausführungsformen der Erfindung finden sich in den Unteransprüchen.

Es wird also erfindungsgemäß vorgeschlagen, als pH-Regulationsmittel die in DE 24 48 471 A1 zwar als vorteilhaft beschriebene, jedoch bei seitherigen Produkten nach diesseitiger Kenntnis nicht verwandte Adipinsäure als pH-Regulationsmittel einzusetzen, und zwar trotz der relativ hohen Schmelztemperatur der Adipinsäure, die eine Aufbringung als Schmelze eher nicht gestattet. Ferner wird vorgeschlagen, der Adipinsäure ein Schmelzpunktsenkungsmittel zuzugeben, was die Herstellung einer Schmelze und die Aufbringung dieser Schmelze prozesstechnisch erleichtert, da deutlich unterhalb der eigentlichen Schmelztemperatur von Adipinsäure gearbeitet werden kann.

Die Schmelztemperatur bezeichnet diejenige Temperatur, bei der ein Phasenübergang eines festen Stoffs, eines Stoffgemischs mit festen Bestandteilen oder eines festen Bestandteils in einem Stoffgemisch in einen flüssigen Aggregatzustand, die Schmelze, erfolgt.

Während die vorgenannte Druckschrift DE 24 48 471 A1 die Kombination von Adipinsäure mit geringen Mengen von Azelainsäure vorschlägt, um die Heißwasserlöslichkeit und damit die Aufbringung in gelöster Form durch Versprühen bewerkstelligen zu können, wobei Azelainsäure weiter die adhesive Kopplung der Adipinsäure an die Windelmaterialien und damit die Depotwirkung verbessert, wurde mit der vorliegenden Erfindung festgestellt, dass sich auf die hier beanspruchte Weise auch die Aufbringung in schmelzflüssiger Form prozesstechnisch vorteilhaft realisieren lässt. Es wird außerdem als vorteilhaft angesehen, dass neben der Adipinsäure keine weiteren Carbonsäuren oder Substanzen mit Säurecharakter eingesetzt werden müssen, wodurch die Komplexität der Steuerung des pH-Werts im Betrieb herabgesetzt wird. Durch die Herabsetzung der Schmelztemperatur kann auch eine Gefährdung von Mitarbeitern in der Fertigung reduziert werden. Ferner wirkt sich dies schonend auf die Herstellungsmaschine und deren Teile aus. Weiter führt eine geringere Prozesstemperatur zu einem schnelleren Erstarren des pH-Regulationsmittels während der Fertigung. Gegenüber einer Aufbringung im gelösten Zustand muss nicht mit verdampfendem Lösungsmittel innerhalb der Fertigungslinie gearbeitet werden. Auch ist die hitzeinduzierte Verdampfung der Adipinsäure selbst und hierdurch hervorgerufene Verfärbung bei Materialien des Produkts reduziert. Auch der Materialverlust selbst kann reduziert werden, und das Produkt selbst ist am Ende des Herstellprozesses ansehnlicher. Weiter führt die Einbringung des pH-Regulationsmittels im schmelzflüssigen Zustand zu einer besseren Depotwirkung, indem die Adipinsäure besser an den Trägermaterialien des Artikels haftet und weniger zu schnellem Auswaschen neigt als Adipinsäure, die in einer gelösten Form appliziert wurde. Dennoch ist aber die Löslichkeit und die Freisetzung bei einer hier in Rede stehenden Benutzungstemperatur, die etwa Körpertemperatur entspricht, als ausreichend gut zu bezeichnen.

Es erweist sich als vorteilhaft, wenn das Schmelzpunktsenkungsmittel Polyethylenglykol oder Glycerin umfasst oder hieraus besteht. Polyethylenglykol und Glycerin senken die Schmelztemperatur von Adipinsäure im pH-Regulationsmittel ab, wodurch eine prozesssichere Verarbeitung möglich ist. Die Verwendung von Polyethylenglykol und Glycerin als Schmelzpunktsenkungsmittel in dem erfindungsgemäßen pH-Regulationsmittel ist möglich, da die Schmelztemperatur der beiden Stoffe niedriger als der Flammpunkt von Adipinsäure ist und der Flammpunkt und die Zersetzungstemperatur von Polyethylenglykol und Glycerin gleichzeitig höher als die Schmelztemperatur von Adipinsäure sind. Dabei ist der Flammpunkt eines Stoffes nach DIN V 14011 diejenige niedrigste Temperatur, bei der sich über einem Stoff ein zündfähiges Dampf-Luft-Gemisch bilden kann. Der Flammpunkt ist auch vom Luftdruck abhängig. Literaturwerte für den Flammpunkt gelten allgemein für einen Luftdruck von 1013 mbar (1 atm, Umgebungsdruck). Die Zersetzungstemperatur beschreibt eine Temperatur, ab der die chemische Zersetzung eines Stoffes beginnt. Dies geschieht im Allgemeinen mittels thermischer Zersetzung durch das Zuführen von Energie in Form von Wärme.

Ferner weisen Polyethylenglykol und Glycerin eine exzellente Biokompatibilität auf, sind also nicht toxisch, was ein Verfahren zur Herstellung eines Inkontinenzartikels vereinfacht und im Gebrauch eine Gefährdung eines Trägers des Inkontinenzartikels ausschließt.

Beispielhaft sind aus dem Stand der Technik physikalische Werte von erfindungsgemäß verwendbarem Polyethylenglykol von der Carl Roth GmbH & Co. KG in der nachfolgenden Tabelle angegeben:
Flammpunkt [°C] Schmelz-temperatur [°C] Aggregatzustand bei RT Zersetzungs-temperatur [°C] Wasserlöslichkeit

| | | | | |
|---|---|---|---|---|
| PEG1000 | 240-270 | 35-40 | Fest 360 | 500 g/l bei 20 °C |
| PEG1500 | 240-270 | 44-48 | Fest 360 | 500 g/l bei 20 °C |
| PEG2000 | 240-270 | 48-52 | Fest 360 | 500 g/l bei 20 °C |

Beispielhaft sind aus dem Stand der Technik physikalische Werte von Glycerin (Glycerin ≥99% von Carl Roth GmbH & Co. KG, Artikelnummer 6967, Daten aus Sicherheitsdatenblatt Version 4.0 de) angegeben:
- Flüssig bei Raumtemperatur, Schmelzpunkt 18-20 °C
- Zersetzungstemperatur >290 °C
- Flammpunkt 199 °C bei 1013 hPa
- In jedem Verhältnis mischbar mit Wasser
- Hygroskopisch
- pH-Wert 6,5-8,5 (in wässriger Lösung: 500 g/l, 20 °C)

Beispielhaft sind aus dem Stand der Technik physikalische Werte von Adipinsäure (Adipinsäure ≥99,5% von Carl Roth GmbH & Co. KG, Artikelnummer 4475, Daten aus Sicherheitsdatenblatt Version 2.0 de) angegeben:
- Fest bei Raumtemperatur, Schmelzpunkt 150-156 °C
- Flammpunkt 196 °C (c.c.)
- pH-Wert 2,7 (in wässriger Lösung: 23 g/l, 25 °C)
- Wasserlöslichkeit 23 g/l bei 25 °C (ECHA)

In einer vorteilhaften Ausführungsform weist das pH-Regulationsmittel eine Schmelztemperatur von 125 °C - 140 °C, insbesondere 125 °C - 135 °C auf.

Nach einer Ausführungsform der Erfindung hat es sich als vorteilhaft erwiesen, wenn das pH-Regulationsmittel Adipinsäure und als Schmelzpunktsenkungsmittel Polyethylenglykol umfasst, mit der Maßgabe, dass die Anteile von Adipinsäure und Polyethylenglykol derart sind, dass ein Verhältnis der Anzahl der OH-Gruppen von Polyethylenglykol zur Anzahl der COOH-Gruppen der Adipinsäure bei höchstens 0,5, insbesondere höchstens 0,4, insbesondere höchstens 0,3, insbesondere höchstens 0,2, insbesondere höchstens 0,1 liegt.

Nach einer anderen Ausführungsform der Erfindung hat es sich als vorteilhaft erwiesen, wenn das pH-Regulationsmittel Adipinsäure und als Schmelzpunktsenkungsmittel Glycerin umfasst, mit der Maßgabe, dass die Anteile von Adipinsäure und Glycerin derart sind, dass ein Verhältnis der Anzahl der OH-Gruppen von Glycerin zur Anzahl der COOH-Gruppen der Adipinsäure bei höchstens 3,0, insbesondere bei höchstens 2,5, insbesondere bei höchstens 2,0, insbesondere bei höchstens 1,5 liegt.

Die genannten Verhältnisse der Anzahl der OH-Gruppen von Polyethylenglykol bzw. Glycerin zur Anzahl der COOH-Gruppen der Adipinsäure erweisen sich als vorteilhaft im Hinblick auf eine Vermeidung einer Esther-Bildung beim Erschmelzen des pH-Regulationsmittels.

In einer vorteilhaften Ausführungsform weist das Polyethylenglykol ein mittleres Molekulargewicht größer 600 g/mol, insbesondere von mindestens 800 g/mol, insbesondere mindestens 1000 g/mol, insbesondere mindestens 1200 g/mol, insbesondere mindestens 1400 g/mol, insbesondere mindestens 1600 g/mol, insbesondere mindestens 1800 g/mol, insbesondere mindestens 2000 g/mol und höchstens 4000 g/mol, insbesondere höchstens 3500 g/mol, insbesondere höchstens 3000 g/mol, insbesondere höchstens 2500 g/mol auf.

Polyethylenglykol ist mit einem mittleren Molekulargewicht größer als 600 g/mol bei Raumtemperatur fest. Somit ist es möglich das pH-Regulationsmittel umfassend Polyethylenglykol und Adipinsäure im schmelzflüssigen Zustand auf eine Komponente des Inkontinenzartikels zu applizieren und dort ortsfest zu verankern. Beim Verfestigen des pH-Regulationsmittels nach dem Applizieren lässt sich feststellen, dass das Polyethylenglykol die Adipinsäure teilweise verkapselt. Im Gebrauch des Inkontinenzartikels wird die Adipinsäure durch Miktion nach und nach freigegeben (Depotwirkung). Polyethylenglykole mit einem höheren Molekulargewicht sind besser wasserlöslich als Polyethylenglykole mit niedrigerem Molekulargewicht. Eine bessere Wasserlöslichkeit unterstützt die Freisetzung der Adipinsäure. Darüber hinaus weist Polyethylenglykol mit höherem Molekulargewicht eine niedrigere Hygroskopizität auf, wodurch die Gefahr eines unerwünschten Eintrags von Feuchtigkeit in den Inkontinenzartikel vermindert ist. Jedoch sollte das mittlere Molekulargewicht des Polyethylenglykols nicht zu hoch sein, da mit steigendem Molekulargewicht auch die Härte des festen Polyethylenglykols steigt. Dies kann zu einem beeinträchtigten Tragekomfort des Inkontinenzartikels führen.

Das mittlere Molekulargewicht wird auch zur Bezeichnung in den Produktspezifikationen angegeben. Beispielsweise wird ein Polyethylenglykol mit einem mittleren Molekulargewicht von 2000 g/mol mit PEG2000 gekennzeichnet.

Polyethylenglykole (chemische Formel: HO-[CH2-CH2-O]n-H) werden durch Polymerisation von Ethylenoxid hergestellt und weisen eine monomerische Grundeinheit (chemische Formel: (-CH2-CH2-O-)) mit einem Molekulargewicht von 44 g/mol auf. Daher entspricht das Molekulargewicht eines Polyethylenglykols dem Molekulargewicht der Grundeinheit multipliziert mit der Anzahl der Grundeinheiten (auch Polymerisationsgrad) plus dem Molekulargewicht von Wasser. Die Polymerisationsreaktion wird gestoppt, wenn der gewünschte Polymerisationsgrad erreicht ist. Wie dem Fachmann bekannt, weist dabei nicht jedes einzelne Polymermolekül dieselbe Anzahl an Grundeinheiten auf. Stattdessen handelt es sich bei den im Handel erhältlichen Polyethylenglykolen üblicherweise um eine Mischung von Polymermolekülen mit geringen Unterschieden im jeweiligen Polymerisationsgrad. Beispielsweise können die Molekulargewichte der einzelnen Polymermoleküle eines im Handel erhältlichen Polyethylenglykols in einem Bereich um das angegebene mittlere Molekulargewicht von ±10% liegen. So weist ein beispielhaftes PEG2000 (Carl Roth GmbH & Co. KG, Artikelnummer 0154, Sicherheitsdatenblatt Version 2.1 de) ein Molekulargewicht ("Molmasse") von 1800-2200 g/mol auf.

Im Rahmen dieser Erfindung ist die Angabe des mittleren Molekulargewichts von Polyethylenglykol so zu verstehen, dass wenigstens 90 Gew.-% des Polyethylenglykols mit einem Molekulargewicht von ±10 % bezogen auf das mittlere Molekulargewicht vorliegen.

Es ist vorteilhaft, wenn das Polyethylenglykol eine Schmelztemperatur von wenigstens 40 °C, vorzugsweise wenigstens 42 °C und einen Flammpunkt von wenigstens 153 °C, insbesondere wenigstens 160 °C aufweist. Eine Schmelztemperatur von wenigstens 40 °C ist vorteilhaft, damit das pH-Regulationsmittel nicht allein beim Anlegen bzw. Tragen des Inkontinenzartikels durch die Körpertemperatur des Trägers erweicht oder gar erschmilzt. Das Polyethylenglykol ist somit im trockenen Inkontinenzartikel im Gebrauch fest und schließt die Adipinsäure teilweise ein, sodass eine Depotwirkung des pH-Regulationsmittel unterstützt wird. Außerdem verbleibt das pH-Regulationsmittel nach dem Applizieren ortsfest auf dem Inkontinenzartikel, und das Trockenheitsgefühl des Trägers beim Anlegen des frischen Produkts ist nicht beeinträchtigt.

Es ist vorteilhaft, wenn das Schmelzpunktsenkungsmittel im Wesentlichen wasserfrei ist und höchstens 5 Gew-% Wasser, insbesondere weniger als 3 Gew-% oder insbesondere kein Wasser enthält. Bei bis zu 5 Gew-% Wasser im Schmelzpunktsenkungsmittel wird das Wasser im Zuge des Erschmelzens des pH-Regulationsmittels verdampft, was in dieser geringen Menge keine Probleme im Prozess verursacht.

Es erweist sich als vorteilhaft, wenn eine Menge an Adipinsäure wenigstens 0,3 g, insbesondere wenigstens 0,5 g, insbesondere wenigstens 0,7 g oder insbesondere wenigstens 0,9 g und höchstens 3,0 g, insbesondere höchstens 2,5 g, insbesondere höchstens 2,0 g, insbesondere höchstens 1,5 g oder insbesondere höchstens 1,0 g je Inkontinenzartikel beträgt.

Bei einer Ausführungsform hat es sich als vorteilhaft erwiesen, wenn ein Flächengewicht der Adipinsäure im Applizierungsbereich wenigstens 1 g/m², insbesondere wenigstens 5 g/m², insbesondere wenigstens 10 g/m², insbesondere wenigstens 15 g/m² oder insbesondere wenigstens 20 g/m², und höchstens 140 g/m², insbesondere höchstens 120 g/m², insbesondere höchstens 100 g/m² oder insbesondere höchstens 80 g/m² beträgt.

Es ist weiter vorteilhaft, wenn eine Schmelztemperatur oder eine Zersetzungstemperatur eines Materials der wenigstens einen Komponente des Inkontinenzartikels, die das pH-Regulationsmittel aufweist, mindestens 50 °C oberhalb einer Applizierungstemperatur des pH-Regulationsmittels liegt. Dadurch, dass eine Schmelztemperatur oder Zersetzungstemperatur der wenigstens einen Komponente mindestens 50 °C oberhalb der Applizierungstemperatur des pH-Regulationsmittels liegt, wird einer Schädigung der Komponente vorgebeugt.

Weiter ist es vorteilhaft, wenn die Schmelztemperatur eines Materials der wenigstens einen Komponente des Inkontinenzartikels, die das pH-Regulationsmittel aufweist, zwischen 230 °C und 280 °C liegt.

Bei einer Ausführungsform des erfindungsgemäßen Inkontinenzartikels erweist es sich als vorteilhaft, wenn eine Flüssigkeitsaufnahme- und -verteilerschicht auf Vliesbasis vorhanden ist, wobei die Flüssigkeitsaufnahme- und -verteilerschicht synthetische oder natürliche Fasern oder eine Kombination aus beiden umfasst, oder daraus besteht. Hierdurch wird in an sich bekannter Weise eine rasche Flüssigkeitsaufnahme - auch bei schwallartiger Flüssigkeitsbeaufschlagung - und Zwischenspeicherung und anschließende gleichmäßige Weiterleitung an Absorptionskörperbereiche zur dauerhaften Speicherung ermöglicht.

Es hat sich als vorteilhaft erwiesen, wenn ein Flächengewicht der Flüssigkeitsaufnahme- und - verteilerschicht zwischen 15 und 50 g/m² liegt.

Es erweist sich als vorteilhaft, wenn das pH-Regulationsmittel auf eine dem Absorptionskörper zugewandte Seite der Flüssigkeitsaufnahme- und -verteilerschicht appliziert ist. Die Adipinsäure wird im Gebrauch mit eintretenden Körperflüssigkeiten bei Miktion, aus dem pH-Regulationsmittel in Richtung auf den Absorptionskörper, also von der Topsheetseite weg, geleitet und sammelt sich nicht in der Nähe der Haut des Benutzers an. Somit wird ein Rückstau saurer Flüssigkeiten zur Topsheetseite reduziert.

Nach einer Ausführungsform des erfindungsgemäßen Inkontinenzartikels erweist es sich als vorteilhaft, wenn der Absorptionskörper zumindest in einer an die Flüssigkeitsaufnahme- und -verteilerschicht angrenzenden Absorptionskörperlage mindestens einen Kanal aufweist, welcher frei von absorbierendem Material ist und somit der raschen Flüssigkeitsaufnahme und -verteilung an noch ungenutzte Bereiche des Absorptionskörpers dient. Ein absorbierendes Material des Absorptionskörpers kann insbesondere ein natürliches oder synthetisches Vliesstoffmaterial und/oder ein superabsorbierendes Material zur Aufnahme und dauerhaften Speicherung von flüssigen Körperausscheidungen umfassen.

Es ist weiter vorteilhaft, wenn an mindestens einer den Kanal begrenzenden Fläche das pH-Regulationsmittel zumindest teilweise vorliegt, d.h. appliziert ist. Es kann so an die im Kanal auftretende Flüssigkeit abgegeben werden.

In einer Ausführungsform hat es sich als vorteilhaft erwiesen, wenn der Kanal insbesondere vollständig durch die Flüssigkeitsaufnahme- und Verteilerschicht, die auch mit pH-Regulationsmittel behandelt sein kann, überfangen ist.

Es kann sich auch als vorteilhaft erweisen, wenn das pH-Regulationsmittel zwischen zwei Lagen aus Vliesstoff angeordnet ist, welche zwischen Absorptionskörper und Topsheet angeordnet sind (Sandwich). Es ergibt sich dadurch auf vorteilhafte Weise eine beabstandete Anordnung des pH-Regulationsmittels vom absorbierenden Material des Absorptionskörpers, so dass eine Gefahr einer durch den Säurecharakter verursachten Beeinträchtigung der Absorptionsfähigkeit, insbesondere im Fall eines vorhandenen superabsorbierenden Materials, weiter vermindert wird.

Gegenstand der Erfindung ist auch ein Verfahren zum Herstellen eines erfindungsgemäßen absorbierenden Inkontinenzartikels, wobei auf wenigstens eine Komponente des Inkontinenzartikels ein pH-Regulationsmittel mit Adipinsäure appliziert wird, wobei das pH-Regulationsmittel Adipinsäure und ein Schmelzpunktsenkungsmittel umfasst, und dass das pH-Regulationsmittel erhitzt wird bis die Adipinsäure erschmilzt und in dem insgesamt schmelzflüssigen pH-Regulationsmittel homogen verteilt vorliegt, und dass dann das pH-Regulationsmittel in diesem schmelzflüssigen Zustand auf die wenigstens eine Komponente des absorbierenden Inkontinenzartikels appliziert wird und daran anschließend wieder verfestigt und somit ortsfest verankert wird.

Es ist vorteilhaft, wenn das pH-Regulationsmittel zum Absenken der Schmelztemperatur der Adipinsäure Polyethylenglykol oder Glycerin als Schmelzpunktsenkungsmittel umfasst oder hieraus besteht.

Nach einer Ausführungsform der Erfindung hat es sich als vorteilhaft erwiesen, wenn das pH-Regulationsmittel bei einer Schmelztemperatur von 125 °C - 140 °C, insbesondere von 125 °C - 135 °C erschmolzen wird.

Es erweist sich auch als vorteilhaft, wenn das pH-Regulationsmittel auf eine Applizierungstemperatur, die wenigstens 5 °C, insbesondere wenigstens 10 °C über der Schmelztemperatur des pH-Regulationsmittels liegt, gebracht wird, wobei die Applizierungstemperatur insbesondere wenigstens 130° C oder insbesondere wenigstens 135 °C, und insbesondere höchstens 150 °C, insbesondere höchstens 145°C, insbesondere höchstens 140 °C beträgt, und dann das pH-Regulationsmittel im schmelzflüssigen Zustand auf die wenigstens eine Komponente des Inkontinenzartikels appliziert wird. Die Applizierungstemperatur des pH-Regulationsmittels sollte über der Schmelztemperatur des pH-Regulationsmittels liegen, damit beim Applizieren auf eine Komponente des Inkontinenzartikels das erschmolzene pH-Regulationsmittel nicht zu rasch wieder erstarrt, insbesondere auf oder in einem Auftragungswerkzeug erstarrt, oder bevor es in die Komponente penetrieren konnte. Ferner ist es vorteilhaft, dass die hohe Schmelz- und Applizierungstemperatur keimtötend wirkt und eine hygienische Verarbeitung unterstützt.

Weiter erweist es sich als vorteilhaft, wenn das pH-Regulationsmittel einen Flammpunkt aufweist, der wenigstens 20 °C über einer Applizierungstemperatur des pH-Regulationsmittels liegt. Somit ist sichergestellt, dass das pH-Regulationsmittel bei der Applizierung nicht entflammt und somit eine sichere Herstellung der Inkontinenzartikel gewährleistet werden kann.

Es wird vorgeschlagen, dass das pH-Regulationsmittel jeweils auf die Komponente des Inkontinenzartikels appliziert wird oder auf eine endlos zugeführte Flachmaterialbahn appliziert wird, aus welcher dann die Komponente erhalten wird, oder dass das pH-Regulationsmittel in einem vorausgegangenen Verfahrensschritt auf eine endlose Flachmaterialbahn aufgebracht und diese Flachmaterialbahn in eine Lagerform überführt wurde und aus der Lagerform bereitgestellt wird und hieraus die Komponente des Inkontinenzartikels gebildet wird. Das pH-Regulationsmittels kann also im Zuge der Herstellung des Inkontinenzartikels in einer schnelllaufenden Maschine, also in line, appliziert und erstarrt werden, oder es kann in einem vorgeordneten Schritt auf eine Flachmaterialbahn appliziert werden, aus der dann, gegebenenfalls nach Zwischenlagerung, die jeweilige Komponente erhalten wird.

Das pH-Regulationsmittel kann mittels Sprühauftrag oder Kontaktauftrag, insbesondere Walzenauftrag, flächig oder diskontinuierlich auf die wenigstens eine Komponente des Inkontinenzartikels appliziert werden. Für das Sprühauftragen kann beispielsweise eine an sich bekannte Sprühvorrichtung, die für einen Heißkleber-Auftrag vorgesehen werden kann, verwendet werden. Dabei kann das pH-Regulationsmittel insbesondere kontinuierlich oder getaktet appliziert werden.

Weitere vorteilhafte Weiterbildungen des Verfahrens sind in den Ansprüchen 24 bis 30 und im Anspruch 33 beschrieben.

Weitere Merkmale und Vorteile der Erfindung sind Gegenstand der nachfolgenden Beschreibung und der zeichnerischen Darstellung von Ausführungsbeispielen. Es zeigen:
Fig. 1 Draufsicht auf einen schematisch dargestellten absorbierenden Inkontinenzartikel mit pH-Regulationsmittel;
Fig. 2 Schnittansicht A-A eines Inkontinenzartikels gemäß Fig. 1;
Fig. 3 eine Fig. 1 entsprechende Ansicht mit einem streifenförmig applizierten pH-Regulationsmittel; und
Fig. 4 eine Fig. 1 entsprechende Ansicht mit einem schraubenförmig applizierten pH-Regulationsmittel;

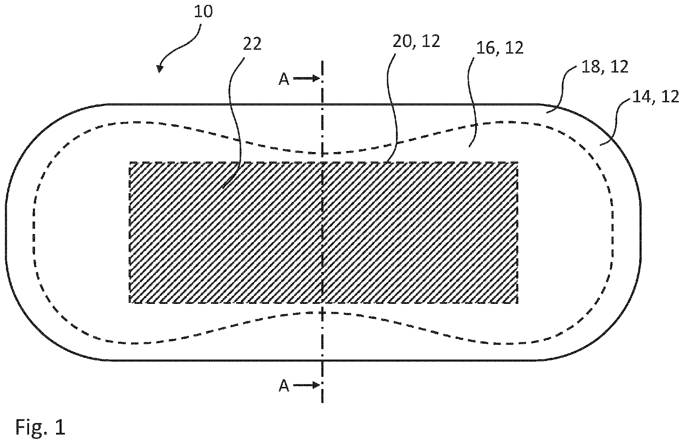

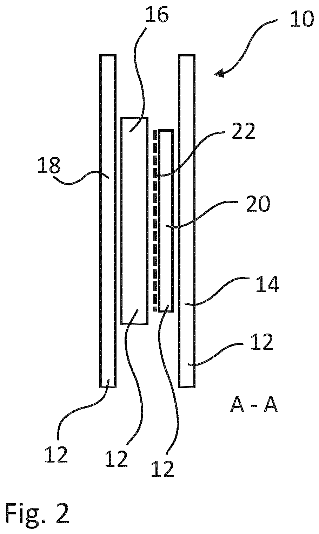

Fig. 1 zeigt eine Draufsicht auf einen schematisch dargestellten absorbierenden Inkontinenzartikel 10 als Einmalprodukt, welches aus mehreren geschichteten Komponenten 12 besteht (siehe auch Schnittansicht A-A der Fig. 2). Der Inkontinenzartikel 10 umfasst ein flüssigkeitsdurchlässiges Topsheet 14, einen darunter angeordneten Absorptionskörper 16 zur Aufnahme und dauerhaften Speicherung von flüssigen Körperausscheidungen und ein darunter angeordnetes und in einem zentralen Bereich flüssigkeitsundurchlässig ausgebildetes Backsheet 18. Zwischen dem Topsheet 14 und dem Absorptionskörper 16 ist vorliegend eine Flüssigkeitsaufnahme- und - verteilerschicht 20 angeordnet. Die Flüssigkeitsaufnahme- und -verteilerschicht 20 weist ein pH-Regulationsmittel 22 auf einer dem Absorptionskörper 16 zugewandten Seite auf.

Das pH-Regulationsmittel 22 wurde im schmelzflüssigem Zustand appliziert und wieder verfestigt. In Fig. 1 ist das Topsheet 14 und die Flüssigkeitsaufnahme- und - verteilerschicht 20 "durchsichtig" dargestellt, um einen Schichtaufbau der einzelnen Komponenten 12 nachvollziehbar abzubilden.

Das pH-Regulationsmittel 22 umfasst Adipinsäure und ein Schmelzpunktsenkungsmittel, welches Polyethylenglykol oder Glycerin umfasst oder daraus besteht. Die Zusammensetzung und das Applizieren des pH-Regulationsmittels 22 ist der obigen Beschreibungseinleitung entnehmbar.

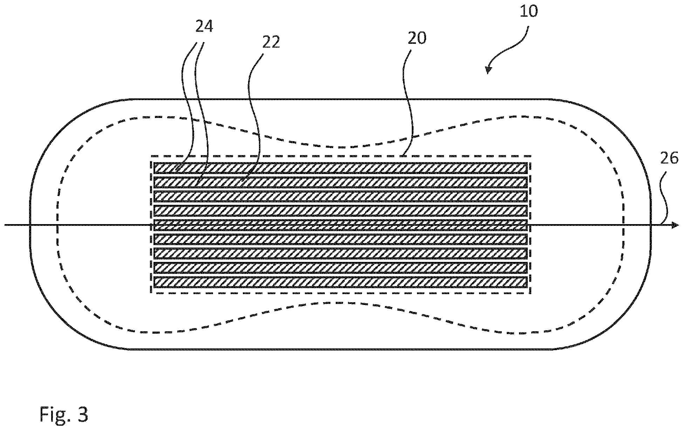

In Fig. 3 ist ein Inkontinenzartikel 10 mit einem auf der Flüssigkeitsaufnahme- und -verteilerschicht 20 streifenförmig applizierten pH-Regulationsmittel 22 dargestellt. Einzelne Streifen 24 des pH-Regulationsmittels 22 erstrecken sich hier beispielhaft parallel zu einer Längsrichtung 26 des Inkontinenzartikels 10 und sind beabstandet zueinander angeordnet. Das Applizieren des pH-Regulationsmittels 22 kann in einem Herstellungsprozess des Inkontinenzartikels 10 mittels Kontaktauftrag, wie zum Beispiel Walzen, oder alternativ mittels Sprühauftrag erfolgen.

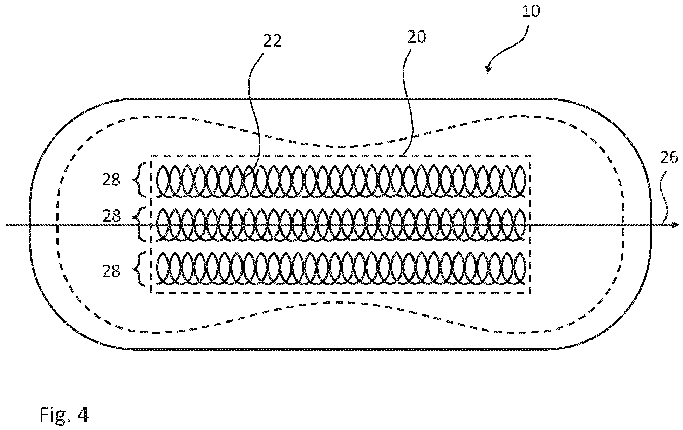

Fig.4 zeigt einen Inkontinenzartikel 10 mit auf der Flüssigkeitsaufnahme- und -verteilerschicht 20 in drei in Längsrichtung 26 erstreckten Bahnen 28 schraubenförmig appliziertem pH-Regulationsmittel 22. Die einzelnen Bahnen 28 verlaufen beispielhaft parallel zu der Längsrichtung 26 des Inkontinenzartikels 10 und sind beispielhaft geringfügig beabstandet zueinander angeordnet.

## Patentansprüche

1. Absorbierender Inkontinenzartikel (10) als Einmalprodukt, umfassend ein flüssigkeitsdurchlässiges Topsheet (14), einen darunter angeordneten Absorptionskörper (16) zur Aufnahme und dauerhaften Speicherung von flüssigen Körperausscheidungen, ein darunter zumindest in einem zentralen Bereich flüssigkeitsundurchlässig ausgebildetes Backsheet (18) und vorzugsweise eine zwischen dem Topsheet (14) und dem Absorptionskörper (16) angeordnete Flüssigkeitsaufnahme- und -verteilerschicht (20), wobei wenigstens eine der vorgenannten Komponenten (12) ein pH-Regulationsmittel (22) mit Adipinsäure aufweist, **dadurch gekennzeichnet,**
**dass** das pH-Regulationsmittel (22) Adipinsäure und ein Schmelzpunktsenkungsmittel umfasst, und dass das pH-Regulationsmittel (22) im schmelzflüssigen Zustand appliziert und wieder verfestigt ist.

2. Inkontinenzartikel (10) nach Anspruch 1, **dadurch gekennzeichnet, dass** das Schmelzpunktsenkungsmittel Polyethylenglykol oder Glycerin umfasst oder hieraus besteht.

3. Inkontinenzartikel (10) nach Anspruch 1 oder 2,
**dadurch gekennzeichnet, dass** das pH-Regulationsmittel (22) eine Schmelztemperatur von 125 °C - 140 °C, insbesondere 125 °C - 135 °C aufweist.

4. Inkontinenzartikel (10) nach Anspruch 2 oder 3,
**dadurch gekennzeichnet, dass** das pH-Regulationsmittel (22) Adipinsäure und als Schmelzpunktsenkungsmittel Polyethylenglykol umfasst, mit der Maßgabe, dass die Anteile von Adipinsäure und Polyethylenglykol derart sind, dass ein Verhältnis der Anzahl der OH-Gruppen von Polyethylenglykol zur Anzahl der COOH-Gruppen der Adipinsäure bei höchstens 0,5, insbesondere höchstens 0,4, insbesondere höchstens 0,3, insbesondere höchstens 0,2, insbesondere höchstens 0,1 liegt.

5. Inkontinenzartikel (10) nach Anspruch 2 oder 3,
**dadurch gekennzeichnet, dass** das pH-Regulationsmittel (22) Adipinsäure und als Schmelzpunktsenkungsmittel Glycerin umfasst, mit der Maßgabe, dass die Anteile von Adipinsäure und Glycerin derart sind, dass ein Verhältnis der Anzahl der OH-Gruppen von Glycerin zur Anzahl der COOH-Gruppen der Adipinsäure bei höchstens 3,0, insbesondere bei höchstens 2,5, insbesondere bei höchstens 2,0, insbesondere bei höchstens 1,5 liegt.

6. Inkontinenzartikel (10) nach einem der Ansprüche 2 bis 4, **dadurch gekennzeichnet, dass** das Polyethylenglykol ein mittleres Molekulargewicht größer 600 g/mol, insbesondere von mindestens 800 g/mol, insbesondere mindestens 1000 g/mol, insbesondere mindestens 1200 g/mol, insbesondere mindestens 1400 g/mol, insbesondere mindestens 1600 g/mol, insbesondere mindestens 1800 g/mol, insbesondere mindestens 2000 g/mol und höchstens 4000 g/mol, insbesondere höchstens 3500 g/mol, insbesondere höchstens 3000 g/mol, insbesondere höchstens 2500 g/mol aufweist.

7. Inkontinenzartikel (10) nach einem der Ansprüche 2 bis 4 oder 6, **dadurch gekennzeichnet, dass** das Polyethylenglykol eine Schmelztemperatur von wenigstens 40 °C, insbesondere wenigstens 42 °C und einen Flammpunkt von wenigstens 153 °C, insbesondere wenigstens 160 °C aufweist.

8. Inkontinenzartikel (10) nach einem der vorstehenden Ansprüche, **dadurch gekennzeichnet, dass** das Schmelzpunktsenkungsmittel im Wesentlichen wasserfrei ist und höchstens 5 Gew.-% Wasser, insbesondere weniger als 3 Gew.-% oder insbesondere kein Wasser enthält.

9. Inkontinenzartikel (10) nach einem der vorstehenden Ansprüche, **dadurch gekennzeichnet, dass** eine Menge an Adipinsäure wenigstens 0,3 g, insbesondere wenigstens 0,5 g, insbesondere wenigstens 0,7 g oder insbesondere wenigstens 0,9 g und höchstens 3,0 g, insbesondere höchstens 2,5 g, insbesondere höchstens 2,0 g, insbesondere höchstens 1,5 g oder insbesondere höchstens 1,0 g je Inkontinenzartikel (10) beträgt.

10. Inkontinenzartikel (10) nach einem der vorstehenden Ansprüche, **dadurch gekennzeichnet, dass** ein Flächengewicht der Adipinsäure im Applizierungsbereich wenigstens 1 g/m², insbesondere wenigstens 5 g/m², insbesondere wenigstens 10 g/m², insbesondere wenigstens 15 g/m² oder insbesondere wenigstens 20 g/m², und höchstens 140 g/m², insbesondere höchstens 120 g/m², insbesondere höchstens 100 g/m² oder insbesondere höchstens 80 g/m² beträgt.

11. Inkontinenzartikel (10) nach einem der vorstehenden Ansprüche, **dadurch gekennzeichnet, dass** die Schmelztemperatur eines Materials der wenigstens einen Komponente (12) des Inkontinenzartikels (10), die das pH-Regulationsmittel (22) aufweist, zwischen 230 °C und 280 °C liegt.

12. Inkontinenzartikel (10) nach einem der vorstehenden Ansprüche, **dadurch gekennzeichnet, dass** eine Flüssigkeitsaufnahme- und -verteilerschicht (20) auf Vliesbasis vorhanden ist, wobei die Flüssigkeitsaufnahme- und -verteilerschicht (20) synthetische oder natürliche Fasern oder eine Kombination aus beiden umfasst, oder daraus besteht.

13. Inkontinenzartikel (10) nach Anspruch 12, **dadurch gekennzeichnet, dass** ein Flächengewicht der Flüssigkeitsaufnahme- und -verteilerschicht (20) zwischen 15 und 50 g/m² liegt.

14. Inkontinenzartikel (10) nach Anspruch 12 oder 13,
**dadurch gekennzeichnet, dass** das pH-Regulationsmittel (22) auf eine dem Absorptionskörper (16) zugewandte Seite der Flüssigkeitsaufnahme- und -verteilerschicht (20) appliziert ist.

15. Inkontinenzartikel (10) nach einem der Ansprüche 12 bis 14, **dadurch gekennzeichnet, dass** der Absorptionskörper (16) zumindest in einer an die Flüssigkeitsaufnahme- und -verteilerschicht (20) angrenzenden Absorptionskörperlage (16) mindestens einen Kanal aufweist, welcher frei von absorbierendem Material ist.

16. Inkontinenzartikel (10) nach Anspruch 15, **dadurch gekennzeichnet, dass** an mindestens einer den Kanal begrenzenden Fläche das pH-Regulationsmittel (22) zumindest teilweise vorliegt.

17. Inkontinenzartikel (10) nach Anspruch 15 oder 16,
**dadurch gekennzeichnet, dass** der Kanal insbesondere vollständig durch die Flüssigkeitsaufnahme- und - verteilerschicht (20) überfangen ist, welche pH-Regulationsmittel (22) umfassen kann.

18. Inkontinenzartikel (10) nach einem der vorstehenden Ansprüche, **dadurch gekennzeichnet, dass** das pH-Regulationsmittel (22) zwischen zwei Lagen aus Vliesstoff angeordnet ist, welche zwischen Absorptionskörper (16) und Topsheet (14) angeordnet sind (Sandwich).

19. Verfahren zum Herstellen eines absorbierenden Inkontinenzartikels (10) nach einem oder mehreren der vorstehenden Ansprüche, wobei auf wenigstens eine Komponente (12) des Inkontinenzartikels (10) ein pH-Regulationsmittel (22) mit Adipinsäure appliziert wird, **dadurch gekennzeichnet, dass** das pH-Regulationsmittel (22) Adipinsäure und ein Schmelzpunktsenkungsmittel umfasst, und dass das pH-Regulationsmittel (22) erhitzt wird bis die Adipinsäure erschmilzt und in dem insgesamt schmelzflüssigen pH-Regulationsmittel (22) homogen verteilt vorliegt, und dass dann das pH-Regulationsmittel (22) in diesem schmelzflüssigen Zustand auf die wenigstens eine Komponente (12) des absorbierenden Inkontinenzartikels (10) appliziert wird und daran anschließend wieder verfestigt und somit ortsfest verankert wird.

20. Verfahren nach Anspruch 19, **dadurch gekennzeichnet, dass** das pH-Regulationsmittel (22) zum Absenken der Schmelztemperatur der Adipinsäure Polyethylenglykol oder Glycerin als Schmelzpunktsenkungsmittel umfasst oder hieraus besteht.

21. Verfahren nach Anspruch 19 oder 20, **dadurch gekennzeichnet, dass** das pH-Regulationsmittel (22) bei einer Schmelztemperatur von 125 C - 140 C, insbesondere von 125 °C - 135 °C erschmolzen wird.

22. Verfahren nach einem der Ansprüche 19 bis 21, **dadurch gekennzeichnet, dass** das pH-Regulationsmittel (22) auf eine Applizierungstemperatur, die wenigstens 5 °C, insbesondere wenigstens 10 °C über der Schmelztemperatur des pH-Regulationsmittels (22) liegt, gebracht wird, wobei die Applizierungstemperatur insbesondere wenigstens 130° C oder insbesondere wenigstens 135 °C, und insbesondere höchstens 150 °C, insbesondere höchstens 145°C, insbesondere höchstens 140 °C beträgt, und dann das pH-Regulationsmittel (22) im schmelzflüssigen Zustand auf die wenigstens eine Komponente (12) des Inkontinenzartikels (10) appliziert wird.

23. Verfahren nach einem der Ansprüche 19 bis 22, **dadurch gekennzeichnet, dass** das pH-Regulationsmittel (22) einen Flammpunkt aufweist, der wenigstens 20 °C über der Applizierungstemperatur des pH-Regulationsmittels (22) liegt.

24. Verfahren nach einem der Ansprüche 20 bis 23, **dadurch gekennzeichnet, dass** das pH-Regulationsmittel (22) Adipinsäure und als Schmelzpunktsenkungsmittel Polyethylenglykol umfasst, mit der Maßgabe, dass die Anteile von Adipinsäure und Polyethylenglykol derart sind, dass ein Verhältnis der Anzahl der OH-Gruppen von Polyethylenglykol zur Anzahl der COOH-Gruppen der Adipinsäure bei höchstens 0,5, insbesondere höchstens 0,4, insbesondere höchstens 0,3, insbesondere höchstens 0,2, insbesondere höchstens 0,1 liegt.

25. Verfahren nach einem der Ansprüche 20 bis 23, **dadurch gekennzeichnet, dass** das pH-Regulationsmittel (22) Adipinsäure und als Schmelzpunktsenkungsmittel Glycerin umfasst, mit der Maßgabe, dass die Anteile von Adipinsäure und Glycerin derart sind, dass ein Verhältnis der Anzahl der OH-Gruppen von Glycerin zur Anzahl der COOH-Gruppen der Adipinsäure bei höchstens 3,0 insbesondere bei höchstens 2,5, insbesondere bei höchstens 2,0, insbesondere bei höchstens 1,5 liegt.

26. Verfahren nach einem der Ansprüche 20 bis 24, **dadurch gekennzeichnet, dass** das Polyethylenglykol ein mittleres Molekulargewicht größer 600 g/mol, insbesondere von mindestens 800 g/mol, insbesondere mindestens 1000 g/mol, insbesondere mindestens 1200 g/mol, insbesondere mindestens 1400 g/mol, insbesondere mindestens 1600 g/mol, insbesondere mindestens 1800 g/mol, insbesondere mindestens 2000 g/mol und höchstens 4000 g/mol, insbesondere höchstens 3500 g/mol, insbesondere höchstens 3000 g/mol, insbesondere höchstens 2500 g/mol aufweist.

27. Verfahren nach einem der Ansprüche 20 bis 24 oder nach Anspruch 26, **dadurch gekennzeichnet, dass** das Polyethylenglykol eine Schmelztemperatur von wenigstens 40 °C, insbesondere wenigstens 42 °C und einen Flammpunkt von wenigstens 153 °C, insbesondere wenigstens 160 °C aufweist.

28. Verfahren nach einem der Ansprüche 19 bis 27, **dadurch gekennzeichnet, dass** das Schmelzpunktsenkungsmittel im Wesentlichen wasserfrei ist und höchstens 5 Gew.-% Wasser, insbesondere weniger als 3 Gew.-% oder insbesondere kein Wasser enthält.

29. Verfahren nach einem der Ansprüche 19 bis 28, **dadurch gekennzeichnet, dass** eine Menge an Adipinsäure wenigstens 0,3 g, insbesondere wenigstens 0,5 g, insbesondere wenigstens 0,7 g oder insbesondere wenigstens 0,9 g und höchstens 3,0 g, insbesondere höchstens 2,5 g, insbesondere höchstens 2,0 g, insbesondere höchstens 1,5 g oder insbesondere höchstens 1,0 g je Inkontinenzartikel (10) beträgt.

30. Verfahren nach einem der Ansprüche 19 bis 29, **dadurch gekennzeichnet, dass** ein Flächengewicht der Adipinsäure im Applizierungsbereich wenigstens 1 g/m², insbesondere wenigstens 5 g/m², insbesondere wenigstens 10 g/m², insbesondere wenigstens 15 g/m² oder insbesondere wenigstens 20 g/m², und höchstens 140 g/m², insbesondere höchstens 120 g/m², insbesondere höchstens 100 g/m² oder insbesondere höchstens 80 g/m² beträgt.

31. Verfahren nach einem der Ansprüche 19 bis 30, **dadurch gekennzeichnet, dass** das pH-Regulationsmittel (22) jeweils auf die Komponente (12) des Inkontinenzartikels (10) appliziert wird oder auf eine endlos zugeführte Flachmaterialbahn appliziert wird, aus welcher die Komponente (12) erhalten wird, oder dass das pH-Regulationsmittel (22) in einem vorausgegangenen Verfahrensschritt auf eine endlose Flachmaterialbahn aufgebracht und diese Flachmaterialbahn in eine Lagerform überführt wurde und aus der Lagerform bereitgestellt wird und hieraus die Komponente (12) des Inkontinenzartikels (10) gebildet wird.

32. Verfahren nach einem der Ansprüche 19 bis 31, **dadurch gekennzeichnet, dass** das pH-Regulationsmittel (22) mittels Sprühauftrag oder Kontaktauftrag, insbesondere Walzenauftrag, flächig oder diskontinuierlich auf die wenigstens eine Komponente (12) des Inkontinenzartikels (10) appliziert wird.

33. Verfahren nach einem der Ansprüche 19 bis 32, dass das pH-Regulationsmittel (22) auf eine Flüssigkeitsaufnahme- und -verteilerschicht (20), insbesondere auf deren dem Absorptionskörper (16) zugewandte Seite, appliziert wird.

## Claims

1. Absorbent incontinence article (10) as a disposable product, comprising a liquid-permeable topsheet (14), an absorbent body (16) arranged thereunder for absorbing and permanently storing liquid body excretions, a backsheet (18) thereunder designed to be liquid-impermeable at least in a central region, and preferably a liquid absorption and distribution layer (20) arranged between the topsheet (14) and the absorbent body (16), at least one of the aforementioned components (12) comprising a pH regulating agent (22) which contains adipic acid, **characterized in that**
the pH regulating agent (22) comprises adipic acid and a melting point lowering agent, and **in that** the pH regulating agent (22) is applied in the molten state and solidified again.

2. Incontinence article (10) according to claim 1, **characterized in that** the melting point lowering agent comprises or consists of polyethylene glycol or glycerin.

3. Incontinence article (10) according to claim 1 or claim 2, **characterized in that** the pH regulating agent (22) has a melting temperature of 125 °C - 140 °C, in particular 125 °C - 135 °C.

4. Incontinence article (10) according to claim 2 or claim 3, **characterized in that** the pH regulating agent (22) comprises adipic acid and, as a melting point lowering agent, polyethylene glycol, with the proviso that the proportions of adipic acid and polyethylene glycol are such that a ratio of the number of OH groups of polyethylene glycol to the number of COOH groups of the adipic acid is at most 0.5, in particular at most 0.4, in particular at most 0.3, in particular at most 0.2, in particular at most 0.1.

5. Incontinence article (10) according to claim 2 or claim 3, **characterized in that** the pH regulating agent (22) comprises adipic acid and, as a melting point lowering agent, glycerin, with the proviso that the proportions of adipic acid and glycerin are such that a ratio of the number of OH groups of glycerin to the number of COOH groups of the adipic acid is at most 3.0, in particular at most 2.5, in particular at most 2.0, in particular at most 1.5.

6. Incontinence article (10) according to any of claims 2 to 4, **characterized in that** the polyethylene glycol has an average molecular weight greater than 600 g/mol, in particular of at least 800 g/mol, in particular at least 1000 g/mol, in particular at least 1200 g/mol, in particular at least 1400 g/mol, in particular at least 1600 g/mol, in particular at least 1800 g/mol, in particular at least 2000 g/mol and at most 4000 g/mol, in particular at most 3500 g/mol, in particular at most 3000 g/mol, in particular at most 2500 g/mol.

7. Incontinence article (10) according to any of claims 2 to 4 or claim 6, **characterized in that** the polyethylene glycol has a melting temperature of at least 40 °C, in particular at least 42 °C and a flash point of at least 153 °C, in particular at least 160 °C.

8. Incontinence article (10) according to any of the preceding claims, **characterized in that** the melting point lowering agent is substantially anhydrous and contains at most 5 wt.% water, in particular less than 3 wt.% or in particular no water.

9. Incontinence article (10) according to any of the preceding claims, **characterized in that** an amount of adipic acid is at least 0.3 g, in particular at least 0.5 g, in particular at least 0.7 g or in particular at least 0.9 g and at most 3.0 g, in particular at most 2.5 g, in particular at most 2.0 g, in particular at most 1.5 g or in particular at most 1.0 g per incontinence article (10).

10. Incontinence article (10) according to any of the preceding claims, **characterized in that** a weight per unit area of adipic acid in the application region is at least 1 g/m², in particular at least 5 g/m², in particular at least 10 g/m², in particular at least 15 g/m² or in particular at least 20 g/m², and at most 140 g/m², in particular at most 120 g/m², in particular at most 100 g/m² or in particular at most 80 g/m².

11. Incontinence article (10) according to any of the preceding claims, **characterized in that** the melting temperature of a material of the at least one component (12) of the incontinence article (10) that comprises the pH regulating agent (22) is between 230 °C and 280 °C.

12. Incontinence article (10) according to any of the preceding claims, **characterized in that** a nonwoven-based liquid absorption and distribution layer (20) is present, the liquid absorption and distribution layer (20) comprising or consisting of synthetic or natural fibers or a combination of the two.

13. Incontinence article (10) according to claim 12,
**characterized in that** a weight per unit area of the liquid absorption and distribution layer (20) is between 15 and 50 g/m².

14. Incontinence article (10) according to claim 12 or claim 13, **characterized in that** the pH regulating agent (22) is applied to a side of the liquid absorption and distribution layer (20) facing the absorption body (16).

15. Incontinence article (10) according to any of claims 12 to 14, **characterized in that** the absorption body (16) has, at least in an absorption body layer (16) adjacent to the liquid absorption and distribution layer (20), at least one channel which is free of absorbent material.

16. Incontinence article (10) according to claim 15,
**characterized in that** the pH regulating agent (22) is at least partially present on at least one surface delimiting the channel.

17. Incontinence article (10) according to claim 15 or claim 16, **characterized in that** the channel is in particular completely overlaid by the liquid absorption and distribution layer (20) which can comprise pH regulating agent (22).

18. Incontinence article (10) according to any of the preceding claims, **characterized in that** the pH regulating agent (22) is arranged between two layers of nonwoven fabric which are arranged between the absorption body (16) and the topsheet (14) (sandwich).

19. Method for producing an absorbent incontinence article (10) according to one or more of the preceding claims, a pH regulating agent (22) which contains adipic acid being applied to at least one component (12) of the incontinence article (10), **characterized in that** the pH regulating agent (22) comprises adipic acid and a melting point lowering agent, and **in that** the pH regulating agent (22) is heated until the adipic acid melts and is homogeneously distributed in the overall molten pH regulating agent (22), and **in that** the pH regulating agent (22) is then applied in this molten state to the at least one component (12) of the absorbent incontinence article (10), and is then solidified again and thus anchored in place thereon.

20. Method according to claim 19, **characterized in that** the pH regulating agent (22), for lowering the melting temperature of the adipic acid, comprises or consists of polyethylene glycol or glycerin as a melting point lowering agent.

21. Method according to claim 19 or claim 20, **characterized in that** the pH regulating agent (22) is melted at a melting temperature of 125 °C - 140 °C, in particular 125 °C - 135 °C.

22. Method according to any of claims 19 to 21,
**characterized in that** the pH regulating agent (22) is brought to an application temperature which is at least 5 °C, in particular at least 10 °C above the melting temperature of the pH regulating agent (22), the application temperature being in particular at least 130 °C or in particular at least 135 °C, and in particular at most 150 °C, in particular at most 145 °C, in particular at most 140 °C, and then the pH regulating agent (22) is applied in the molten state to the at least one component (12) of the incontinence article (10).

23. Method according to any of claims 19 to 22,
**characterized in that** the pH regulating agent (22) has a flash point which is at least 20 °C above the application temperature of the pH regulating agent (22).

24. Method according to any of claims 20 to 23,
**characterized in that** the pH regulating agent (22) comprises adipic acid and, as a melting point lowering agent, polyethylene glycol, with the proviso that the proportions of adipic acid and polyethylene glycol are such that a ratio of the number of OH groups of polyethylene glycol to the number of COOH groups of the adipic acid is at most 0.5, in particular at most 0.4, in particular at most 0.3, in particular at most 0.2, in particular at most 0.1.

25. Method according to any of claims 20 to 23,
**characterized in that** the pH regulating agent (22) comprises adipic acid and, as a melting point lowering agent, glycerin, with the proviso that the proportions of adipic acid and glycerin are such that a ratio of the number of OH groups of glycerin to the number of COOH groups of the adipic acid is at most 3.0, in particular at most 2.5, in particular at most 2.0, in particular at most 1.5.

26. Method according to any of claims 20 to 24,
**characterized in that** the polyethylene glycol has an average molecular weight greater than 600 g/mol, in particular of at least 800 g/mol, in particular at least 1000 g/mol, in particular at least 1200 g/mol, in particular at least 1400 g/mol, in particular at least 1600 g/mol, in particular at least 1800 g/mol, in particular at least 2000 g/mol and at most 4000 g/mol, in particular at most 3500 g/mol, in particular at most 3000 g/mol, in particular at most 2500 g/mol.

27. Method according to any of claims 20 to 24 or claim 26, **characterized in that** the polyethylene glycol has a melting temperature of at least 40 °C, in particular at least 42 °C, and a flash point of at least 153 °C, in particular at least 160 °C.

28. Method according to any of claims 19 to 27,
**characterized in that** the melting point lowering agent is substantially anhydrous and contains at most 5 wt.% water, in particular less than 3 wt.% or in particular no water.

29. Method according to any of claims 19 to 28,
**characterized in that** an amount of adipic acid is at least 0.3 g, in particular at least 0.5 g, in particular at least 0.7 g or in particular at least 0.9 g and at most 3.0 g, in particular at most 2.5 g, in particular at most 2.0 g, in particular at most 1.5 g or in particular at most 1.0 g per incontinence article (10).

30. Method according to any of claims 19 to 29,
**characterized in that** a weight per unit area of adipic acid in the application region is at least 1 g/m², in particular at least 5 g/m², in particular at least 10 g/m², in particular at least 15 g/m² or in particular at least 20 g/m², and at most 140 g/m², in particular at most 120 g/m², in particular at most 100 g/m² or in particular at most 80 g/m².

31. Method according to any of claims 19 to 30,
**characterized in that** the pH regulating agent (22) is applied to each component (12) of the incontinence article (10) individually or is applied to an endlessly fed flat material web from which the component (12) is obtained, or **in that** the pH regulating agent (22) has been applied in a previous method step to an endless flat material web, and this flat material web has been transferred into a storage form and is provided from the storage form, and the component (12) of the incontinence article (10) is formed therefrom.

32. Method according to any of claims 19 to 31,
**characterized in that** the pH regulating agent (22) is applied by spray application or contact application, in particular roller application, over the surface or discontinuously to the at least one component (12) of the incontinence article (10).

33. Method according to any of claims 19 to 32,
**characterized in that** the pH regulating agent (22) is applied to a liquid absorption and distribution layer (20), in particular to its side facing the absorption body (16).

## Revendications

1. Article absorbant pour incontinence (10) en tant que produit à usage unique, comprenant une feuille supérieure (14) perméable aux liquides, un corps absorbant (16) disposé en dessous de celle-ci et destiné à absorber et à stocker durablement des excrétions corporelles liquides, une feuille arrière (18) située là-dessous et conçue pour être imperméable aux liquides au moins dans une zone centrale, et de préférence une couche d'absorption et de distribution de liquides (20) disposée entre la feuille supérieure (14) et le corps absorbant (16), dans lequel au moins l'un des composants (12) susmentionnés comprend un agent régulateur de pH (22) avec de l'acide adipique, **caractérisé par le fait**
**que** l'agent régulateur de pH (22) comprend de l'acide adipique et un agent abaissant le point de fusion, et que l'agent régulateur de pH (22) est appliqué à l'état fondu et est resolidifié.

2. Article d'incontinence (10) selon la revendication 1, **caractérisé par le fait que** l'agent abaissant le point de fusion comprend ou est constitué de polyéthylène glycol ou de glycérine.

3. Article d'incontinence (10) selon la revendication 1 ou 2, **caractérisé par le fait que** l'agent régulateur de pH (22) présente une température de fusion de 125 °C à 140 °C, en particulier de 125 °C à 135 °C.

4. Article d'incontinence (10) selon la revendication 2 ou 3, **caractérisé par le fait que** l'agent régulateur de pH (22) comprend de l'acide adipique et, comme agent abaissant le point de fusion, du polyéthylène glycol, à condition que les proportions d'acide adipique et de polyéthylène glycol soient telles qu'un rapport du nombre des groupes OH du polyéthylène glycol au nombre des groupes COOH de l'acide adipique soit de 0,5 tout au plus, en particulier de 0,4 tout au plus, en particulier de 0,3 tout au plus, en particulier de 0,2 tout au plus, en particulier de 0,1 tout au plus.

5. Article d'incontinence (10) selon la revendication 2 ou 3, **caractérisé par le fait que** l'agent régulateur de pH (22) comprend de l'acide adipique et, comme agent abaissant le point de fusion, de la glycérine, à condition que les proportions d'acide adipique et de glycérine soient telles qu'un rapport du nombre des groupes OH de la glycérine au nombre des groupes COOH de l'acide adipique soit de 3,0 tout au plus, en particulier de 2,5 tout au plus, en particulier de 2,0 tout au plus, en particulier de 1,5 tout au plus.

6. Article d'incontinence (10) selon l'une quelconque des revendications 2 à 4, **caractérisé par le fait que** le polyéthylène glycol présente un poids moléculaire moyen supérieur à 600 g/mol, en particulier d'au moins 800 g/mol, en particulier d'au moins 1000 g/mol, en particulier d'au moins 1200 g/mol, en particulier d'au moins 1400 g/mol, en particulier d'au moins 1600 g/mol, en particulier d'au moins 1800 g/mol, en particulier d'au moins 2000 g/mol et de 4000 g/mol tout au plus, en particulier de 3500 g/mol tout au plus, en particulier de 3000 g/mol tout au plus, en particulier de 2500 g/mol tout au plus.

7. Article d'incontinence (10) selon l'une quelconque des revendications 2 à 4 ou 6, **caractérisé par le fait que** le polyéthylène glycol présente une température de fusion d'au moins 40 °C, en particulier d'au moins 42 °C et un point d'éclair d'au moins 153 °C, en particulier d'au moins 160 °C.

8. Article d'incontinence (10) selon l'une quelconque des revendications précédentes, **caractérisé par le fait que** l'agent abaissant le point de fusion est pour l'essentiel anhydre et contient tout au plus 5 % en poids d'eau, en particulier moins de 3 % en poids ou en particulier ne contient pas d'eau.

9. Article d'incontinence (10) selon l'une quelconque des revendications précédentes, **caractérisé par le fait qu'**une quantité d'acide adipique est d'au moins 0,3 g, en particulier d'au moins 0,5 g, en particulier d'au moins 0,7 g ou en particulier d'au moins 0,9 g et de 3,0 g tout au plus, en particulier de 2,5 g tout au plus, en particulier de 2,0 g tout au plus, en particulier de 1,5 g tout au plus ou en particulier de 1,0 g tout au plus par article d'incontinence (10).

10. Article d'incontinence (10) selon l'une quelconque des revendications précédentes, **caractérisé par le fait qu'**un grammage de l'acide adipique dans la zone d'application est d'au moins 1 g/m², en particulier d'au moins 5 g/m², en particulier d'au moins 10 g/m², en particulier d'au moins 15 g/m² ou en particulier d'au moins 20 g/m², et de 140 g/m² tout au plus, en particulier de 120 g/m² tout au plus, en particulier de 100 g/m² tout au plus ou en particulier de 80 g/m² tout au plus.

11. Article d'incontinence (10) selon l'une quelconque des revendications précédentes, **caractérisé par le fait que** la température de fusion d'un matériau dudit au moins un composant (12) de l'article d'incontinence (10), qui comprend l'agent régulateur de pH (22) est comprise entre 230 °C et 280 °C.

12. Article d'incontinence (10) selon l'une quelconque des revendications précédentes, **caractérisé par le fait qu'**une couche d'absorption et de distribution de liquides (20) à base de non-tissé est présente, dans lequel ladite couche d'absorption et de distribution de liquides (20) comprend ou est constituée de fibres synthétiques ou naturelles ou d'une combinaison des deux.

13. Article d'incontinence (10) selon la revendication 12, **caractérisé par le fait qu'**un grammage de la couche d'absorption et de distribution de liquides (20) est compris entre 15 et 50 g/m².

14. Article d'incontinence (10) selon la revendication 12 ou 13, **caractérisé par le fait que** l'agent régulateur de pH (22) est appliqué sur une face de la couche d'absorption et de distribution de fluide (20), qui montre vers le corps absorbant (16).

15. Article d'incontinence (10) selon l'une quelconque des revendications 12 à 14, **caractérisé par le fait que** le corps absorbant (16) présente au moins un canal qui est exempt de matériau absorbant, au moins dans une couche de corps absorbant (16) adjacente à la couche d'absorption et de distribution de liquides (20).

16. Article d'incontinence (10) selon la revendication 15, **caractérisé par le fait que** l'agent régulateur de pH (22) est au moins partiellement présent sur au moins une face délimitant le canal.

17. Article d'incontinence (10) selon la revendication 15 ou 16, **caractérisé par le fait que** le canal est en particulier entièrement recouvert par la couche d'absorption et de distribution de liquides (20) qui peut comprendre de l'agent régulateur de pH (22).

18. Article d'incontinence (10) selon l'une quelconque des revendications précédentes, **caractérisé par le fait que** l'agent régulateur de pH (22) est disposé entre deux couches de non-tissé qui sont disposées entre (sandwich) le corps absorbant (16) et la feuille supérieure (14).

19. Procédé de fabrication d'un article absorbant pour incontinence (10) selon une ou plusieurs des revendications précédentes, dans lequel un agent régulateur de pH (22) comprenant de l'acide adipique est appliqué sur au moins un composant (12) de l'article absorbant pour incontinence (10), **caractérisé par le fait que** l'agent régulateur de pH (22) comprend de l'acide adipique et un agent abaissant le point de fusion, et **par le fait que** l'agent régulateur de pH (22) est chauffé jusqu'à ce que l'acide adipique fonde et soit réparti de manière homogène dans l'agent régulateur de pH (22) fondu dans l'ensemble, et **par le fait que** l'agent régulateur de pH (22) est ensuite appliqué à l'état fondu sur ledit au moins un composant (12) de l'article absorbant pour incontinence (10) et est ensuite solidifié à nouveau sur celui-ci et ainsi fermement ancré à celui-ci.

20. Procédé selon la revendication 19, **caractérisé par le fait que**, pour abaisser la température de fusion de l'acide adipique, l'agent régulateur de pH (22) comprend ou est constitué de polyéthylène glycol ou de glycérine comme agent abaissant le point de fusion.

21. Procédé selon la revendication 19 ou 20, **caractérisé par le fait que** l'agent régulateur de pH (22) est fondu à une température de fusion de 125 °C à 140 °C, en particulier de 125 °C à 135 °C.

22. Procédé selon l'une quelconque des revendications 19 à 21, **caractérisé par le fait que** l'agent régulateur de pH (22) est porté à une température d'application qui est supérieure d'au moins 5 °C, en particulier d'au moins 10 °C à la température de fusion de l'agent régulateur de pH (22), dans lequel la température d'application est en particulier d'au moins 130 °C ou en particulier d'au moins 135 °C, et en particulier de 150 °C tout au plus, en particulier de 145 °C tout au plus, en particulier de 140 °C tout au plus, et puis l'agent régulateur de pH (22) est appliqué à l'état fondu sur ledit au moins un composant (12) de l'article d'incontinence (10).

23. Procédé selon l'une quelconque des revendications 19 à 22, **caractérisé par le fait que** l'agent régulateur de pH (22) présente un point d'éclair qui est au moins 20 °C supérieur à la température d'application de l'agent régulateur de pH (22).

24. Procédé selon l'une quelconque des revendications 20 à 23, **caractérisé par le fait que** l'agent régulateur de pH (22) comprend de l'acide adipique et, comme agent abaissant le point de fusion, du polyéthylène glycol, à condition que les proportions d'acide adipique et de polyéthylène glycol soient telles qu'un rapport du nombre des groupes OH du polyéthylène glycol au nombre des groupes COOH de l'acide adipique soit de 0,5 tout au plus, en particulier de 0,4 tout au plus, en particulier de 0,3 tout au plus, en particulier de 0,2 tout au plus, en particulier de 0,1 tout au plus.

25. Procédé selon l'une quelconque des revendications 20 à 23, **caractérisé par le fait que** l'agent régulateur de pH (22) comprend de l'acide adipique et, comme agent abaissant le point de fusion, de la glycérine, à condition que les proportions d'acide adipique et de glycérine soient telles qu'un rapport du nombre des groupes OH de la glycérine au nombre des groupes COOH de l'acide adipique soit de 3,0 tout au plus, en particulier de 2,5 tout au plus, en particulier de 2,0 tout au plus, en particulier de 1,5 tout au plus.

26. Procédé selon l'une quelconque des revendications 20 à 24, **caractérisé par le fait que** le polyéthylène glycol présente un poids moléculaire moyen supérieur à 600 g/mol, en particulier d'au moins 800 g/mol, en particulier d'au moins 1000 g/mol, en particulier d'au moins 1200 g/mol, en particulier d'au moins 1400 g/mol, en particulier d'au moins 1600 g/mol, en particulier d'au moins 1800 g/mol, en particulier d'au moins 2000 g/mol et de 4000 g/mol tout au plus, en particulier de 3500 g/mol tout au plus, en particulier de 3000 g/mol tout au plus, en particulier de 2500 g/mol tout au plus.

27. Procédé selon l'une quelconque des revendications 20 à 24 ou selon la revendication 26, **caractérisé par le fait que** le polyéthylène glycol présente une température de fusion d'au moins 40 °C, en particulier d'au moins 42 °C et un point d'éclair d'au moins 153 °C, en particulier d'au moins 160 °C.

28. Procédé selon l'une quelconque des revendications 19 à 27, **caractérisé par le fait que** l'agent abaissant le point de fusion est pour l'essentiel anhydre et contient tout au plus 5 % en poids d'eau, en particulier moins de 3 % en poids ou en particulier ne contient pas d'eau.

29. Procédé selon l'une quelconque des revendications 19 à 28, **caractérisé par le fait qu'**une quantité d'acide adipique est d'au moins 0,3 g, en particulier d'au moins 0,5 g, en particulier d'au moins 0,7 g ou en particulier d'au moins 0,9 g et de plus 3,0 g tout au plus, en particulier de 2,5 g tout au plus, en particulier de 2,0 g tout au plus, en particulier de 1,5 g tout au plus ou en particulier de 1,0 g tout au plus par article d'incontinence (10).

30. Procédé selon l'une quelconque des revendications 19 à 29, **caractérisé par le fait qu'**un grammage de l'acide adipique dans la zone d'application est d'au moins 1 g/m², en particulier d'au moins 5 g/m², en particulier d'au moins 10 g/m², en particulier d'au moins 15 g/m² ou en particulier d'au moins 20 g/m², et de 140 g/m² tout au plus, en particulier de 120 g/m² tout au plus, en particulier de 100 g/m² tout au plus ou en particulier de 80 g/m² tout au plus.

31. Procédé selon l'une quelconque des revendications 19 à 30, **caractérisé par le fait que** l'agent régulateur de pH (22) est appliqué respectivement sur le composant (12) de l'article d'incontinence (10) ou est appliqué sur une bande de matériau plat amenée en continu, à partir de laquelle le composant (12) est obtenu, ou **par le fait que** l'agent régulateur de pH (22) est appliqué sur une bande de matériau plat sans fin dans une étape de procédé précédente et que cette bande de matériau plat a été mise dans une forme de stockage et est fournie à partir de la forme de stockage et le composant (12) de l'article d'incontinence (10) est formé à partir de celle-ci.

32. Procédé selon l'une quelconque des revendications 19 à 31, **caractérisé par le fait que** l'agent régulateur de pH (22) est appliqué par pulvérisation ou par contact, en particulier au rouleau, de manière plane ou discontinue sur ledit au moins un composant (12) de l'article d'incontinence (10).

33. Procédé selon l'une quelconque des revendications 19 à 32, **caractérisé par le fait que** l'agent régulateur de pH (22) est appliqué sur une couche d'absorption et de distribution de liquides (20), en particulier sur sa face tournée vers le corps d'absorption (16).
